# EUROPEAN PATENT SPECIFICATION

(11) **EP 2 505 128 B1**
(45) Date of publication and mention of the grant of the patent: **08.05.2019**
(21) Application number: 12002316.3
(22) Date of filing: 30.03.2012
(51) Int. Cl.: A61B 3/12, A61B 3/13, A61B 3/15, A61B 3/10, A61B 3/00

(54) **Ophthalmic photographing apparatus**
Ophthalmische Fotografievorrichtung
Appareil photographique ophtalmique

(30) Priority: 31.03.2011 JP 2011080751
(43) Date of publication of application: 03.10.2012
(73) Proprietor: Nidek co., Ltd., Gamagori Aichi (JP)
(72) Inventor: Shibata, Naohisa, Gamagori Aichi (JP); Hanebuchi, Masaaki, Gamagori Aichi (JP)
(74) Representative: Hoefer & Partner Patentanwälte mbB

(56) References cited:
- US-A1- 2008 151 189
- US-A1- 2010 277 692
- ZAWADZKI ROBERT J ET AL: "Cellular resolution volumetric in vivo retinal imaging with adaptive optics-optical coherence tomography.", OPTICS EXPRESS 2 MAR 2009 LNKD- PUBMED:19259248, vol. 17, no. 5, 2 March 2009 (2009-03-02), pages 4084-4094, XP002681397, ISSN: 1094-4087

## Description

### BACKGROUND

The present invention relates to an ophthalmic photographing apparatus that assists in observing a patient's eye and photographs the eye.

Conventionally, there has been proposed an ophthalmic photographing apparatus that photographs a fundus at a magnification as high as cellular-level magnification by two-dimensionally scanning a patient's eye with laser light and receiving the laser light reflected therefrom (US2010277692). Specifically, the apparatus includes: a photographing unit that photographs a fundus image of the patient's eye; a high-magnification photographing unit that obtains a fundus image at a magnification higher than that of the fundus image photographed by the photographing unit; and a wavefront compensation section that eliminates aberration of the patient's eye through the high-magnification photographing unit. By using the wavefront compensation section, it is possible to eliminate aberration from the high-magnification fundus image photographed by the high-magnification photographing unit, and it is possible to observe the fundus at the cellular level.

However, since the high-magnification photography image which is photographed by the above-mentioned apparatus can be obtained by photographing a local portion of the fundus, in order to obtain information necessary for a diagnostic or a test and in order to check the fundus in a certain wide area, it is necessary to perform photographing a plurality of times.

Further, in a case of photographing of the local portion of the patient's eye for an image other than the high-magnification photography image, in order to detect the entirety thereof, photographing may be performed a plurality of times. However, although a region necessary for a diagnostic or a test is photographed, a plurality of photography images should be detected in a state where they are associated with each other.

### SUMMARY

According to the present invention, in an ophthalmic photographing apparatus that photographs a local portion of the patient's eye such as the anterior eye part or the fundus, provided is an ophthalmic photographing apparatus capable of sufficiently acquiring the photography images necessary for a diagnostic or a test and thus finding association between photography images which are acquired.

The invention is set out in the appended claims.

### BRIEF DESCRIPTION OF THE DRAWINGS

Fig. 1 is an explanatory diagram illustrating an optical system of an ophthalmic photographing apparatus.
Fig. 2 is a block diagram illustrating a control system of the ophthalmic photographing apparatus.

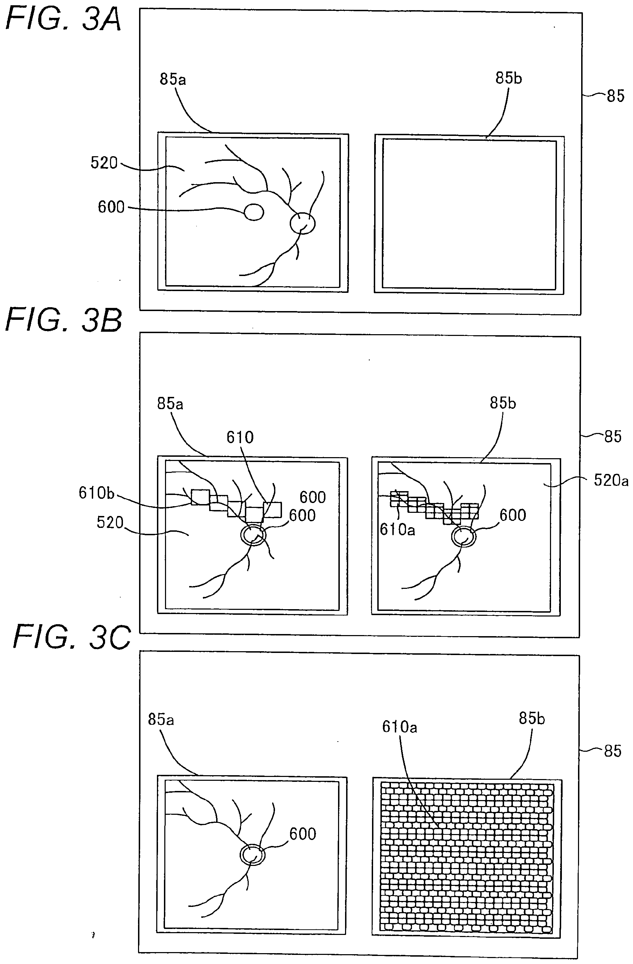

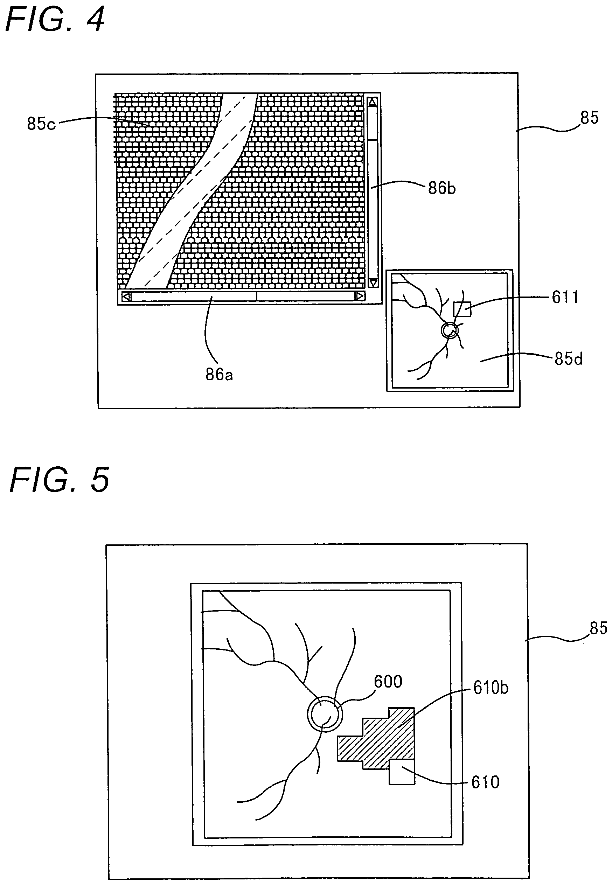

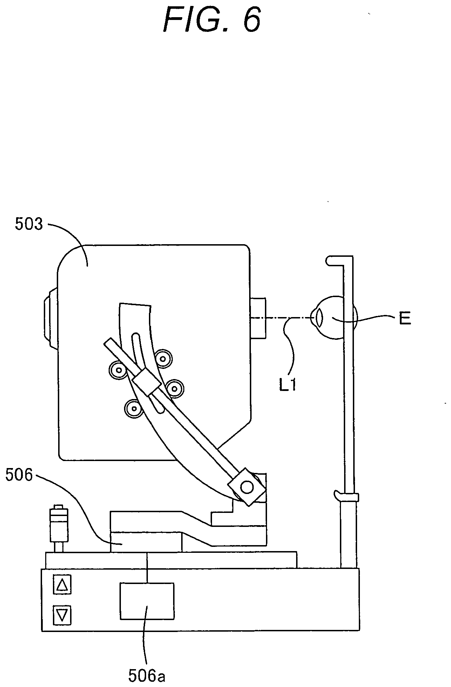

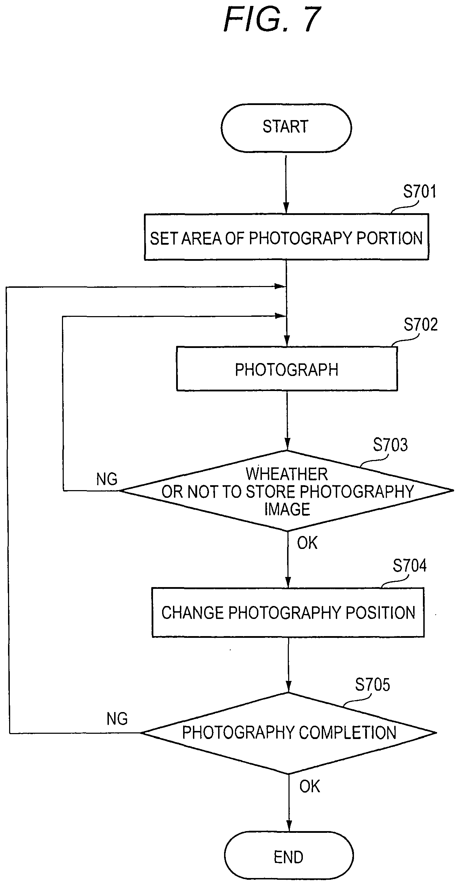

Fig. 3A is a diagram illustrating an example of a display screen of a monitor.Fig. 3B is a diagram illustrating an example of the display screen of the monitor.Fig. 3C is a diagram illustrating an example of the display screen of the monitor.Fig. 4 is a diagram illustrating an example of a panorama image which is displayed on the monitor.Fig. 5 is a diagram illustrating a changed pattern of the display screen of the monitor.Fig. 6 is a schematic external view of the ophthalmic photographing apparatus.Fig. 7 is a flowchart of processing steps of continuously photographing first photography images.

### DESCRIPTION

According to the present invention, in the case of photographing the local portion of the patient's eye such as the anterior eye part or the fundus, it is possible to sufficiently acquire photography images necessary for a diagnostic or confirmation. Further, it is possible to detect association between photography images which are acquired.

An embodiment of the present invention will be described with reference to drawings. Here, description will be given of an example of an ophthalmic photographing apparatus that photographs a fundus at a magnification as high as cellular-level magnification by two-dimensionally scanning a patient's eye with laser light and receiving the laser light reflected therefrom. Note that, the high-magnification photography image described in the embodiment is defined as an image which is photographed at a magnification to some extent that it is possible to check body tissues such as a cornea and a retina, microvessels, and the like at the cellular level. On the other hand, a photography image with a wide angle of view is defined as an image, which is photographed at a magnification to some extent that it is possible to check or observe the entire image of the prescribed portion of the fundus, such as an anterior eye part image or a front image of the fundus which is photographed by a general fundus camera or the like, and an image which is photographed in a wider area than the high-magnification photography image.

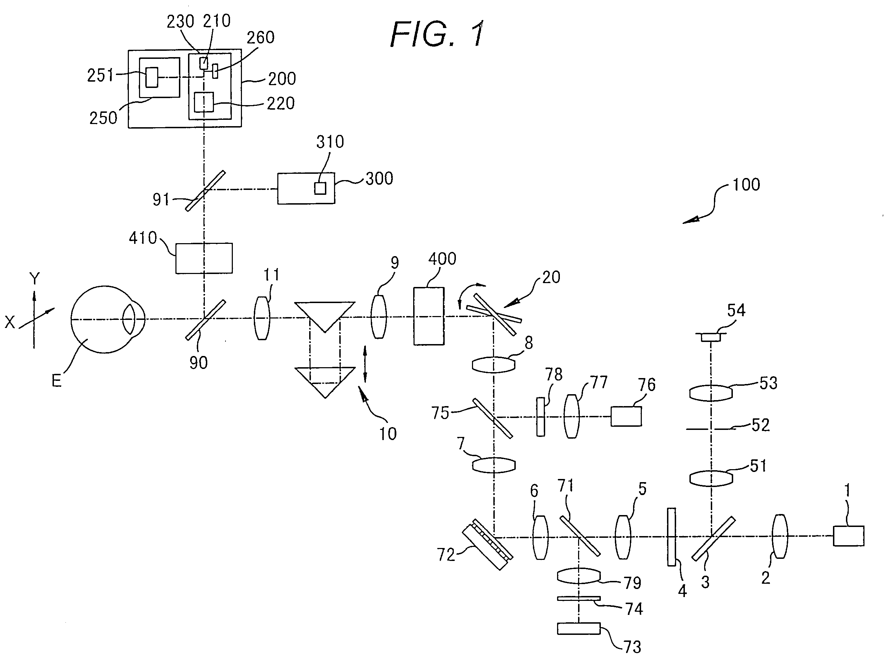

Fig. 1 shows an optical system of the ophthalmic photographing apparatus according to the embodiment.

The ophthalmic photographing apparatus includes: a first photographing unit (high-magnification photographing unit) 100 that photographs a fundus of an examinee's eye E at a high magnification (high resolution); a second photographing unit 200 that obtains a fundus image (hereinafter referred to as a second photography image) which has a wider area than the first photography image in order to specify a photography position of the high-magnification fundus image (hereinafter referred to as a first photography image); and a tracking unit (positional deviation correction optical system) 300 that detects a time-varying change in positional deviation caused by involuntary eye movement of the patient's eye E and obtains information on the movement position thereof.

Note that, the first photographing unit 100 includes a wavefront compensation section that compensates low-order aberration and high-order aberration of the patient's eye E. The second photographing unit 200 has a view angle (a wide view angle) at which the fundus is observed in a relatively wide area in order to find the position of the fundus on the first photography image, while the first photographing unit 100 has a view angle (a narrow view angle) which is made narrower than that of the second photographing unit 200.

The first photographing unit 100 includes: a first illumination optical system that illuminates the fundus of the patient's eye E with illumination light (illumination rays); a first photographing optical system that receives the light (reflected rays) reflected from the fundus and obtains the first photography image; and the wavefront compensation section that detects wavefront aberration of the patient's eye E and compensates the wavefront aberration of the patient's eye E. For example, the first photographing unit 100 has a configuration of a scanning laser ophthalmoscope that uses a confocal optical system.

The first illumination optical system includes a light source 1 (first light source) that emits near-infrared illumination light, a lens 2, a beam splitter 3 constituted of a half mirror and the like, a polarizing plate 4, a lens 5, a beam splitter 71, a lens 6, a wavefront compensation device 72, a lens 7, and a beam splitter 75. The first illumination optical system further includes a lens 8, the scanning section 20 that scans the fundus with the illumination light (spot light) which is emitted from the light source 1, a deflecting unit 400 that corrects a scanning area of the illumination light in the scanning section 20 on the basis of the detection result of a tracking unit 300, a lens 9, a visibility-degree correction unit 10 constituted of two prisms, a lens 11, and a beam splitter 90 that makes optical axes of the second photographing unit 200 and other units substantially coaxial with the optical axis of the first illumination optical system.

As the light source 1, a SLD (Super Luminescent Diode) light source or the like is used. Besides, as the light source 1, a light source, which emits highly-converged spot light, may be used, and a semiconductor laser or the like may be used. The scanning section 20 includes: a resonant mirror (resonant scanner) that scans the fundus with the illumination light in the vertical direction (Y direction) in order to obtain the high-magnification photography image; a galvano mirror (galvano scanner) that functions as a deflection member which deflects the illumination light toward the fundus in a horizontal direction (X direction) in order to determine the scanning position of the resonant scanner on the fundus. Each mirror is placed at a pupil conjugate position of the patient's eye. Note that, in a case where the resonant mirror is driven in the horizontal direction (X direction), the direction of the galvano mirror may be set to the vertical direction (Y direction). Besides, the scanning position of the fundus may be determined by setting the direction of the galvano mirror to the XY directions.

The deflecting section 400 includes two galvano mirrors. The deflecting section 400 deflects the illumination light, which passes through the scanning section 20, by a predetermined amount in the horizontal and vertical directions, and corrects the scanning area of the scanning section 20 on the basis of the position correction information (to be described later) which is obtained by detection of the tracking unit 300. The deflecting section 400 is placed at the pupil conjugate position similarly to the scanning section 20.

Note that, in the above description, the scanning of the spot light is performed by two galvano mirrors of the scanning section 20, but the deflecting section 400 may perform both determination of the scanning position and correction of the scanning area.

Note that, the configuration in which the high-magnification photography image is acquired is not limited to the above description, and it may be possible to adopt a configuration in which the high-magnification photography image can be acquired in the prescribed area of the fundus (a photography area which is narrower than the photography area of the second photography image). For example, as the light source 1, a line laser, which emits linear rays extending in the horizontal or vertical direction relative to the fundus, is used, and the scanning section 20 is provided with the galvano mirror which is driven in a direction orthogonal to the rays. With such a configuration, by performing one-dimensional scanning in the direction orthogonal to the rays emitted from the light source 1, the high-magnification photography image may be acquired.

The illumination light emitted from the light source 1 is made into parallel light by the lens 2, passes through the beam splitter 3, and then the illumination light is made to have only an S-polarized light component by the polarizing plate 4. The illumination light after passing through the polarizing plate 4 is once collected by the lens 5, passes through the beam splitter 71, is made into parallel light by the lens 6, and enters the wavefront compensation device 72. The illumination light reflected by the wavefront compensation device 72 is relayed through the lens 7 and the lens 8, and heads for the scanning section 20.

The illumination light after passing through the scanning section 20 passes through the deflecting section 400, is collected again by the lens 9, passes through the visibility-degree correction unit 10, the lens 11 and the beam splitter 90, and is collected on the fundus of the patient's eye E, whereby the fundus is two-dimensionally scanned with the illumination light by the scanning section 20. Note that, by moving one prism of the visibility-degree correction unit 10 in the arrow directions shown in the drawing, its optical path length is changed, thereby correcting the degree of visibility. In addition, the beam splitter 90 constituted of a dichroic mirror and the like reflects the rays from the tracking unit 300 and the second photographing unit 200 to be described later, and transmits the rays from the light source 1 and a light source 76 to be described later. Note that, exit ends of the light source 1 and the light source 76 have a conjugate relationship to the patient's eye E. In such a manner, the first illumination optical system, which two-dimensionally illuminates the fundus with illumination light, is formed. In addition, the light source 1 and the light source 76 may be formed as a single light source instead of being separately provided.

Next, a configuration of the first photographing optical system will be described. The first photographing optical system shares the optical path from the beam splitter 90 to the beam splitter 3 with the first illumination optical system, and further includes a lens 51, a pinhole plate 52 that is placed at a position conjugate to the fundus, a collecting lens 53, and a light receiving element 54. The pinhole plate 52 has a pinhole that is made coaxial with the fundus. For example, as the light receiving element 54, an APD (avalanche photodiode) or the like may be used.

The reflection light, which is reflected from the fundus illuminated by the light source 1, travels on the optical path in the reverse direction to the first illumination optical system, only the S-polarized light is transmitted by the polarizing plate 4, and thereafter a part of the light is reflected by the beam splitter 3. The reflection light comes into focus on the pinhole of the pinhole plate 52 through the lens 51. The reflection light coming in focus on the pinhole is received on the light receiving element 54 through the lens 53. Note that, while a part of the illumination light is reflected from the cornea of the eye, most of the light is removed by the pinhole plate 52. Thereby, the light reflected from the cornea has a reduced adverse effect on the obtained image. In such a manner, the first photographing optical system is formed, and the photography image photographed by the first photographing optical system is made into the first photography image.

Note that, deflection angles (swing angles) of the mirrors of the scanning section 20 are determined such that a view angle of the fundus image obtained by the first photographing unit 100 is equal to a predetermined angle. For example, when a predetermined area of the fundus is observed and photographed at a high magnification (here, observed and photographed at a cellular level), a photography area of the first photographing image is set to an area of about 300 to 1500 µm.

Next, a configuration of the wavefront compensation section (compensation optical system) will be described. The wavefront compensation section includes a wavefront sensor 73, a polarizing plate 74, the light source 76, a lens 77, a polarizing plate 78, and a lens 79. The wavefront compensation section shares the optical members from the beam splitter 71 to the beam splitter 90 with the first illumination optical system. The wavefront sensor 73 includes a microlens array which is formed of multiple microlenses, and a two-dimensional light receiving element which receives the rays transmitted through the microlens array.

Further, the light source 76 (a second light source) used in detecting aberration is formed as a light source that emits light within an infrared wavelength range different from that of the light source 1, and a laser diode or the like is used as the light source 76. The laser light emitted from the light source 76 is made into parallel light by the lens 77, made into polarized light (P-polarized light) by the polarizing plate 78 so as to have a polarization direction perpendicular to the polarization direction of the illumination light emitted from the light source 1, and guided to the optical path of the first illumination optical system by the beam splitter 75. Note that, the conjugate position of the fundus is located between the lenses 7 and 8, and the exit end of the light source 76 has a conjugate relationship to the conjugate position of the fundus. Here, a half mirror is used as the beam splitter 75.

The laser light reflected by the beam splitter 75 is collected on the fundus of the patient's eye E through the optical path of the first illumination optical system. In this case, the polarization direction of the light reflected from the cornea is kept constant, and the light is made into P-polarized light. On the other hand, since the light reflected from the fundus is made into scattered light, the polarization state of a part of the reflected light is inverted, and the reflected light is made into S-polarized light. The light reflected from the fundus and the cornea is reflected by the wavefront compensation device 72 through the optical members of the first illumination optical system, deflected from the optical path of the first illumination optical system by the beam splitter 71, and then guided to the wavefront sensor 73 through the lens 79 and the polarizing plate 74 that transmits only S-polarized light component. The polarizing plate 74 blocks the light (P-polarized light) having the polarization direction of the light of the second light source illuminating the fundus (that is, blocks the P-polarized light reflected from the cornea and the fundus), transmits the polarized light component (S-polarized light) having the polarization direction perpendicular to that of the P-polarized light, and guides the light to the wavefront sensor 73.

The beam splitter 71 has a property of transmitting the light with a wavelength of the light source 1 and reflecting the light with a wavelength of the light source 76 for aberration detection. Thereby, the wavefront sensor 73 detects the light having the S-polarized light component of the light scattered from the fundus, and does not detect the light reflected from the cornea and the optical elements. Note that, the scanning section 20, the reflection surface of the wavefront compensation device 72, and the microlens array of the wavefront sensor 73 are made to be substantially conjugate to the pupil of the patient's eye. Further, a light receiving surface of the wavefront sensor 73 is made to be substantially conjugate to the fundus of the patient's eye E. As the wavefront sensor 73, it may be possible to use not only a Hartmann-Shack detector, a wavefront curvature sensor that detects change in light intensity, or the like, but also an element capable of detecting high-order aberration such as wavefront aberration and low-order aberration.

Further, the wavefront compensation device 72 employs, for example, a liquid-crystal spatial phase modulator, and a reflective LCOS (Liquid Crystal On Silicon) or the like may be used. In addition, the wavefront compensation device 72 is disposed to be oriented such that it can compensate aberration of predetermined linearly polarized light (S-polarized light) such as the illumination light (S-polarized light) from the light source 1, the illumination light (S-polarized light) reflected from the fundus, and the reflected light (S-polarized light) of the light for wavefront aberration detection. With such a configuration, the wavefront compensation device 72 modulates the S-polarized light component of the incident light.

The wavefront compensation section (compensation optical system) controls the wavefront compensation device 72 on the basis of the wavefront aberration of the reflected light of the light source 76 from the fundus that is detected by the wavefront sensor 73, so as to thereby remove the wavefront aberration of the illumination light emitted from the light source 1 and the wavefront aberration of the reflected light thereof in addition to the S-polarized light component of the reflected light of the light source 76. Thereby, the wavefront compensation device 72 is able to obtain the first photography image, from which the wavefront aberration is removed (wavefront aberration is compensated), with a high magnification. Here, the rays of the light source 76 and the rays of the light source 1 are set to substantially coincide with each other on the fundus, and aberration of the illumination light of the first photographing unit 100 and aberration of the fundus image at the position of the pinhole plate 52 are removed. In addition, the fundus image obtained by the first photographing unit 100 is stored as the first photography image in a storage unit.

Next, a configuration of the second photographing unit 200 will be described. The second photographing unit 200 is used to perform photographing in a wider area (the second photography image) than the first photographing unit. The obtained second photography image is used to specify and check the position of the first photography image. Note that, it is preferable that the second photographing unit 200 should be capable of obtaining the fundus image of the patient's eye E with a wide area (for example, a view angle of about 20 to 60 degrees) for observation in real time. An observation/photographing system of an existing fundus camera, or an optical system and a control system of an existing scanning laser ophthalmoscope (SLO) are used therefor. Hereinafter, for convenience of description, the optical system, and the like are shown by a block diagram.

The second photographing unit 200 includes a second illumination optical system 230 that two-dimensionally illuminates the fundus, a second photographing optical system 250 that photographs the fundus image, and a fixation target provision section 260 that fixes the patient's eye. The second illumination optical system 230 includes a second light source 210 that emits illumination light to illuminate the fundus, and a scanning section 220 that two-dimensionally scans the fundus with the illumination light.

Further, the second photographing optical system 250 includes a light receiving element 251 that receives the light reflected from the fundus which is illuminated by the second illumination optical system 230. The fixation target provision section 260 according to the embodiment has a well-known liquid crystal display as means for providing the fixation target. Then, by switching on and off a plurality of pixels constituting a liquid crystal display, the provided position of the fixation target is changed for the patient's eye. Note that, a control section 82 to be described later is used for the change of the provided position of the fixation target. Besides, the fixation target provision section 260 adopts a well-known configuration capable of guiding the line of sight of the patient's eye.

The optical axis of the second photographing unit 200 is made to be substantially coaxial with the optical axis of the first photographing unit 100 by the beam splitter 90 disposed between the scanning section 220 and the examinee's eye. The angle of the beam splitter 90 is set such that the reflected light from the cornea entering the second photographing unit 200 is reduced. Further, the beam splitter 90 has the property of transmitting the light from the light source 1 and the light source 76, and reflecting the light with wavelengths of the second light source 210 and a light source of the tracking unit 300 to be described later. Furthermore, a beam splitter 91, which is operable to make the optical axis of the second photographing unit 200 coaxial with the optical axis of the tracking unit 300, and a deflecting section 410, which has the same function as the deflecting section 400, are disposed on an optical path between the beam splitter 90 and the second photographing unit 200.

As the second light source 210, a laser diode, which emits laser light within an infrared wavelength range, or the like is used. The scanning section 220 includes mirrors that deflect (reflect) the laser light in the X and Y directions as described above. Here, the deflection angles of the mirrors of the scanning section 220 are set such that a view angle of the fundus image obtained by the second photographing unit 200 is larger than the view angle of the first photographing unit 100. Note that, in the present embodiment, the second photographing unit 200 is configured to be able to perform photographing at a wide view angle (for example, about 20 to 60 degrees) which is enough to obtain characteristic portions of the fundus such as a macular portion and a papilla portion thereof.

The laser light emitted from the second light source 210 is emitted from the second photographing unit 200 through the second illumination optical system 230. The laser light passes through the beam splitter 91 and the deflecting section 410, is reflected by the beam splitter 90, and collected on the fundus of the patient's eye E. The fundus is two-dimensionally scanned with the laser light collected on the fundus by driving of the scanning section 220. The light reflected from the fundus is received on the light receiving element 251 of the second photographing optical system 250, and the fundus image (the second photography image) with the wide view angle is obtained. Note that, the fundus image obtained by the second photographing unit 200 is stored as the second photography image in the storage unit.

In the present embodiment, the fundus illumination is performed such that the fundus is two-dimensionally scanned with the rays by the scanning section 220. However, the fundus illumination may be performed such that the fundus is scanned in a direction perpendicular to the direction of the slit light formed in a line shape. Alternatively, an illumination optical system of the existing fundus camera using a hole mirror and the like may be used.

Next, a configuration of the tracking unit 300 will be described. Usually, eyes continually move finely because of involuntary eye movement. Such fine movement of the eye has a great effect when the fundus is observed and photographed at a high magnification such as a cellular-level magnification, as compared with the case where the fundus is observed in a wide area. For this reason, the tracking unit 300 detects swinging of the eye caused by involuntary eye movement or the like, thereby obtaining information on positional correction that is used for preventing a positional deviation in the first photography image.

The tracking unit 300 includes a light source 310 that emits illumination light within an infrared wavelength range. For example, as the light source 310, a SLD light source or the like is used. The scanning is circularly performed with the illumination light emitted from the light source 310 by driving of a resonant mirror so as to thereby form a tracking indicator (here, the indicator is formed to have a diameter substantially equal to that of the papilla) having a ring shape on the fundus. The light reflected from the fundus is received on the light receiving element. The circular scanning using the illumination light is performed about 100 to 2,000 times per second by the fast driving of the resonant mirror. The illumination light emitted from the tracking unit 300 is made to be substantially coaxial with the optical axis of the second photographing unit 200 by the beam splitter 91, and then made to be substantially coaxial with the optical axis of the first photographing unit 100 by the beam splitter 90 through the deflecting section 410. Note that, the beam splitter 91 has a property of reflecting the light from the light source 310, and transmitting the light from the light source 210.

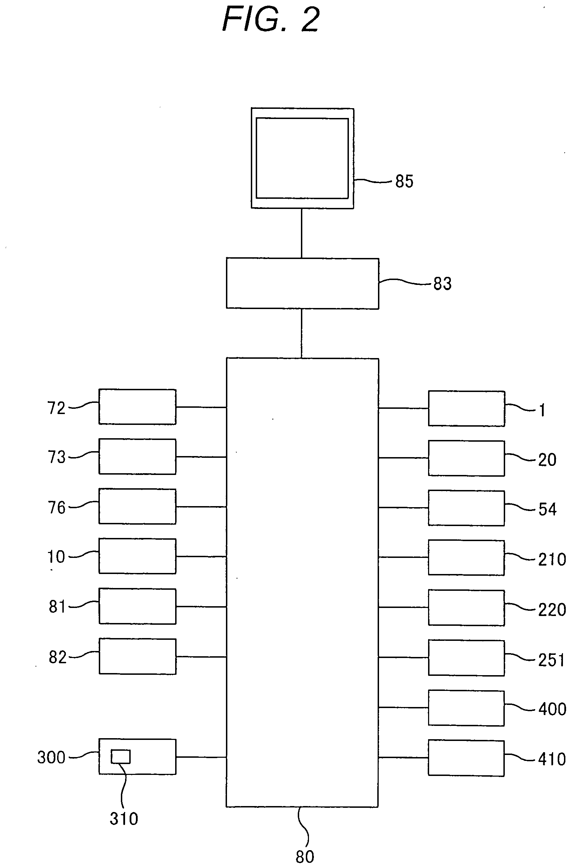

Next, a control system of the ophthalmic photographing apparatus will be described. Fig. 2 is a block diagram illustrating the control system of the ophthalmic photographing apparatus. A control unit 80 performs the driving control of the entire apparatus. The control unit 80 is connected with the light source 1, the visibility-degree correction unit 10, the scanning section 20, the light receiving element 54, the wavefront compensation device 72, the wavefront sensor 73, the light source 76, the light source 210, the scanning section 220, the light receiving element 251, the tracking unit 300, the deflecting section 400, and the deflecting section 410. Further, the control unit 80 is connected with the storage unit 81, a control unit 82 formed of a mouse, a touch panel, or the like, an image processing unit 83, and a monitor 85.

The image processing unit 83 displays the fundus images (the first photography image and the second photography image) with different view angles on the monitor 85 on the basis of the light receiving signals from the light receiving element 54 and the light receiving element 251. Further, the image processing unit 83 displays a mark 610 (refer to Fig. 3B) on the second photography image as an observation image in association with the photography position of the first photography image. Note that, the mark 610 is formed in a shape of which the inner area coincides with the photography area of the first photography image. In the present embodiment, the rectangular mark 610, which represents a photography area, with a predetermined size is displayed. Furthermore, the image processing unit 83 performs image processing capable of stitching a plurality of first photography images to each other at different positions on the fundus image, thereby forming a panoramic image with a wide view angle.

The storage unit 81 stores various kinds of setting information in advance. For example, the storage unit 81 stores, as photographing conditions, a normal mode of separately photographing the first photography images and displaying them and panorama modes (manual panorama mode, automatic panorama mode) of stitching the plurality of first photography images which are successively photographed and checking them at the same time (the high-magnification fundus images are stitched so as to thereby checked as a single photography image with a wide area). Further, the storage unit 81 not only stores the second photography image (still image) but also stores the first photography images in association with the photography position information. By storing the first photography images in association with the photography position information, a plurality of first photography images are appropriately associated with each other. Further, the display positions of the first photography images on the monitor 85 are correctly specified. Furthermore, it is possible to correctly stitch the first photography images to the second photography image which is displayed on the monitor 85.

The monitor 85 displays a moving image of the fundus image (the second photography image as an observation image) which is renewed at a predetermined frame rate (for example, about 10 to 100 Hz). Further, the monitor 85 displays still images of the first photography images and the second photography image stored in the storage unit 81 separately from the observation image. A display example of the monitor 85 will be described later.

The control unit 82 moves the display position of the mark 610 which is formed on the second photography image. The control unit 82 is used for not only a photography area setting section, which sets a local photography position (area) of the fundus photographed by the first photographing unit 100, but also input means for setting various conditions for the photography.

An operation of the ophthalmic photographing apparatus having the above-mentioned configuration will be described. Here, it is assumed that the panorama mode (manual panorama mode) is used. In the panorama mode, by selecting different local areas (photography locations) of the second fundus image, a plurality of first photography images (the fundus images at cellular level) with a narrow view angle are acquired. Then, by stitching the plurality of first photography images through the image processing, even in the case of the high-magnification fundus images, the images can be checked in a wide area.

Fig. 3 shows display examples of the monitor 85 of the present embodiment. The monitor 85 is provided with two display spaces 85a and 85b. The left-side display space 85a displays the second photography image 520 as an observation image and the mark 610, which represents the photography position of the first photography image. The right-side display space 85b displays a second photography image 520a (a still image), which is stored in the storage unit 81, and a first photography images 610a. Note that, each first photography image 610a is displayed as a reduced image such as a thumbnail so as to be superimposed upon the second photography image 520a, or is displayed such that the mark representing the photography position of the first photography image is synthesized with the second photography image 520a. Note that, the display positions of the first photography images are determined on the basis of the photography position information.

Note that, it is assumed herein that the still image of the second photography image 520a acquired first is continuously displayed on the display space 85b. Besides, the display of the display space 85b may be sequentially renewed whenever the still image of the second photography image 520a is photographed. Alternatively, the display space 85b may display the observation image (the moving image) of the second photography image which is photo-received by the light receiving element 251. In this case, the control unit 80 calculates the positions of the first photography images relative to the second photography image (the moving image) on the basis of the photography position information to be described later.

Further, the display space 85b may display the fundus images which are photographed at a view angle substantially the same as that of the second photography image 520a. The substantially similar view angle means a view angle capable of specifying the positions at which the first photography images are stitched, and means a view angle which is wide to some extent that it includes the characteristic portions of the fundus such as optic nerve papilla and the like. For example, a part of the second photography image 520a stored in the memory 81 may be cut out and displayed on the display space 85b in an enlarged manner. Alternatively, for example, when the photography areas of the first photography images are large, the second photography image 520a may be displayed in a reduced manner.

Further, the display space 85b may display not only the photography images of infrared rays but also various kinds of the fundus images such as color photography images and fluorescent photography images which are obtained by fluorescent (FA) photographing. Note that, the photography image is photographed by a separate apparatus.

First, in a state where the patient's eye E is fixed by turning on the fixation target of the fixation target provision optical system 260, a tester aligns the eye E with the apparatus by operating an operation unit (not shown in the drawing) such as a joy stick. At this time, the visibility-degree correction unit 10 is driven through the operation of the control unit 82, and the visibility degree of the patient's eye E is corrected. As shown in Fig. 3A, in the alignment, the moving image of the second photography image 520 obtained by the photographing of the second photographing unit 200 is displayed on the display space 85a. The tester performs the alignment while viewing the second photography image 520 as an observation image.

After alignment ends, when a command signal to operate the tracking unit 300 is input through the operation of the control unit 82, the control unit 80 causes the tracking unit 300 to emit laser light, thereby forming a tracking indicator on the fundus of the patient's eye E (not shown in the drawing). Further, the image processing unit 83 displays a reticle 600 having a shape the same as that of the tracking indicator at a position of the corresponding second photography image 520 (inside the observation screen frame). The tester appropriately moves the apparatus and the fixation lamp while viewing the reticle 600 on the second photography image 520, thereby adjusting positions thereof such that the papilla of the patient's eye E shown in the observation screen frame is superimposed on the reticle 600.

When the papilla of the second photography image 520 is superimposed on the reticle 600 and the command signal to start tracking is input through the operation of the control unit 82, the control unit 80 drives the tracking unit 300 and the deflecting sections 400 and 410, thereby starting tracking.

The tracking unit 300 sends the light receiving result, which is obtained at the time of starting the tracking, to the control unit 80, and thereafter transmits the light receiving result (the light receiving information), which is obtained for each one scanning (whenever the ring of the scanning is formed), to the aberration control unit 80. The control unit 80 compares reference information with the light receiving information subsequently obtained, and drives the deflecting section 410 and the deflecting section 400 in synchronization with each other on the basis of the movement position information so as to obtain the light receiving information which is the same as the reference information (that is, so as to superimpose the entire eye-tracking target on the papilla).

When the tracking is performed, even if the eye E finely moves, the movement is compensated. Hence, the fundus images displayed on the monitor 85 are prevented from moving. Note that, on the basis of the detection result of the positional deviation caused by the involuntary eye movement obtained by the position detection unit 300, through the display control of the image processing unit 83, the display position of the mark 610, which determines the photography position of the first photography image, may be slightly corrected.

Subsequently, the tester photographs the second photography image 520, which is displayed on the display space 85a, and the first photography image at the designated position of the mark 610 on the fundus. First, the tester moves the mark 610, which is displayed on the display space 85a, on the second photography image 520 through the operation of the control unit 82, and adjusts the first photography image to a position (area) at which the tester wants to perform photography. Note that, the mark 610 may be moved by a drag operation using a mouse, and the position to be moved may be directly designated by an operation of the touch panel.

When the photographing signal is input through the operation of the control unit 82, the control unit 80 performs driving control of the scanning section 20 so as to photograph the fundus in the area designated by the mark 610. The illumination position of the illumination light, which illuminates the fundus through the driving of the resonant scanner, is moved through the driving of the galvano mirror, and the illumination light is used in scanning in the area corresponding to the mark 610. Further, the control unit 80 dynamically controls the compensation optical system on the basis of the measurement result of the optical distribution (the light receiving signal), which is obtained by the wavefront sensor 73, through the driving control of the wavefront compensation section. Then, the control unit 80 acquires a still image of the first photography image through the light receiving element 54 in conjunction with the operation of the scanning section 20, associates the acquired first photography image with the photography position information (the position information of the mark 610), and stores the image in the storage unit 81. Further, the control unit 80 obtains the still image of the second photography through the light receiving element 251, and stores the image in the storage unit 81.

On the other hand, the image processing unit 83 stitches the first photography image 610a to the displayed second photography image 520a by performing the display control of the second photography image 520a stored in the storage unit 81. Here, the stitching processing will be described.

The mark 610, which is displayed on the display space 85a, represents the photography area of the first photography image 610a in the second photography image 520. The control unit 80 acquires the coordinate position of the mark 610 for the characteristic portion of the second photography image 520 as the photography position information at timing of photographing the first photography image, associates the first photography image 610a with the photography position information, and stores the image in the storage unit 81.

For example, the characteristic portions of the second photography image 520 include the optic nerve papilla portion and blood vessels. Alternatively, the reticle 610 (center coordinates of the reticle) is stored together with the second photography image 520 in the storage unit 81, and the information can be used in the photography position information. Besides, various kinds of information included in the second photography image 520 can be used in the photography position information.

The image processing unit 83 calls the photography position information and the first photography image 610a stored in the storage unit 81, and determines a position at which the images are stitched to the second photography image 520a acquired first on the basis of the photography position information. At this time, by calculating the correlation with the second photography image, the stitching positions may be adjusted.

The image processing unit 83 forms a thumbnail through the image processing of the acquired first photography image 610a, and synthesizes the first photography image 610a (the thumbnails) at the determined stitching position through the image processing so as to thereby display the image on the display space 85b. Further, the image processing unit 83 synthesizes and displays the photography completion mark 610b, which represents that the photography is completed, on the second photography image 520 of the display space 85a through the image processing. Each photography completion mark 610b has a shape the same as the mark 610, but is displayed in a state where the photography completion mark 610b is changed in color and contour in order to be distinguished from the mark 610. In order to distinguish the mark 610b from the mark 610, thumbnail images may be displayed.

Next, in order to widen the area in which the first photography images are completely photographed, the position of the mark 610 is moved to the photography completion mark 610b through the operation of the control unit 82 so as to be adjacent to the mark 610b, and photographing is performed in the same manner as described above, thereby acquiring the first photography image 610a. The first photography image 610a, which is photographed second, is also stored together with the photography position information in the storage unit 81, and is synthesized with the second photography image 520a of the display space 85b through the processing of the image processing unit 83, whereby the synthesized image is displayed.

Then, the image processing unit 83 stitches the first photography images (the high-magnification fundus images) to the second photography image 520a for each photographing of the first photography image, thereby forming a panoramic image of the first photography images on the display space 85b.

Note that, when two first photography images adjacent to each other partially overlaps with each other, the image processing unit 83 overwrites a common portion on the data of the first photography image subsequently photographed. Alternatively, the common portions (the stitched portions of the fundus images) are averaged. In addition, when the averaging is performed, it can be expected to suppress the effect of noise occurring at the time of photography and thereby improve image quality.

When photographing of the first photography image is performed a plurality of times at the same location, the first photography image, which is most recently obtained, is rewritten in the information, and thus a storage capacity of the storage unit 85 is efficiently used. On the other hand, by sequentially adding the information pieces of the plurality of first photography images through integration processing (addition processing), image quality is improved. Besides, averaging processing of averaging the information pieces of the plurality of first photography images may be performed. Further, among the plurality of first photography images, the selection processing of selecting images with higher correlation between the first photography images adjacent to each other may be performed. In such a manner, the stitching of the first photography images photographed at different positions based on various kinds of calculations is repeated.

Furthermore, when the tracking unit 300 that tracks fine movement of the eye is provided, a processing, in which the first photography image photographed when the visual fixation is unstable is not used in synthesis of panorama, may be performed. In this case, it is possible to further accurately form a panoramic image.

Further, in the above-mentioned description, the tester separately designates the position of the mark 610 through the operation of the control unit 82, thereby changing the photography position of the first photography image (the manual panorama mode). Besides, in selection of the photography position of the first photography image, there are various methods.

For example, through the operation of the control unit 82, the photography area of the first photography image is set to be larger than the imaging view angle of each first photography image (for example, a photography area is selected so as to involve a lesion area). Then, the control unit 80 automatically (continuously) moves the photography position of the first photography image within the designated photography area (the automatic panorama mode). In such a manner, the photographing of the first photography image is easily performed a plurality of times through one setting of the control unit 82 as a photography area setting section.

Fig. 7 shows a flowchart of processing steps in a case of continuously photographing first photography images. First, in step S701, the photography area of the first photography image is selected through the operation of the control unit 82 as the photography area setting section. Note that, the photography area is set to be an area larger than the view angle of each first photography image. Next, in step S702, the control unit 80 performs photographing at the photography position indicated by the mark 610 within the photography area. Note that, the photography position indicated by the mark 610 is automatically set by the control unit 80. Subsequently, in step S703, the control unit 80 determines whether to store the photography image. For example, the control unit 80 calculates prescribed correlation between common portions of the photography images adjacent to each other, and if there is prescribed correlation, then the photography images are stored in the storage unit 81 in the processing step not shown in the drawing, and the processing advances to step S704. In addition, in step S703, if it is determined that the photographing is performed first within the photography area, the processing advances to step S704. On the other hand, in step S703, if the prescribed correlation is not established between photography images, the processing returns to step S702, and photographing is repeated at the same location. When the processing advance to step S704, the control unit 80 moves the photography position indicated by the mark 610 to the next photography position. Then, in step S705, the control unit 80 determines whether or not the photographing is completed. For example, when the photography position indicated by the mark 610 is out of the photography area, it is determined that the photographing is completed, and thus the photographing operation ends. On the other hand, when the photography position indicated by the mark 610 is within the photography area, it is determined that the photographing is not completed. Thus, the processing returns to step S702, the operations from step S702 to S705 are repeated.

Further, in the automatic panorama mode, photographing may be performed a plurality of times (for example, 10 times) at the same photography position. In this case, the control unit 80 selects images, which are used to form the panoramic image, among the plurality of first photography images, which are acquired at the same photography position, after the photographing is completed (or photographing at the same location is completed). Alternatively, the first photography image, which has highest correlation with an adjacent image, may be selected through the selection processing. Alternatively, as described above, a panoramic image is formed through a prescribed calculation processing.

When the first photography image 610a (thumbnail) synthesized and displayed on the display space 85b is selected by the operation of the control unit 82, as shown in Fig. 3C, the first photography image 610a is displayed as a fundus image having an original size on the display space 85b in an enlarged manner. Then, when the first photography image displayed in an enlarged manner is repeatedly selected, as shown in Fig. 3B, a display state, which shows photography history of the first photography image, is recovered.

Further, when the first photography image 610a displayed in an enlarged manner is displayed on the display section 85, a checking mark for checking a position thereof may be formed on the display space 85a (observation screen) such that the photography position of the first photography image can be checked. Such a checking mark is formed by the image processing unit 83 on the basis of the photography position information corresponding to the selected first photography image 610a.

Further, when wanting to check the entire panoramic image, the tester changes the display of the monitor 85 to a panorama display screen through the operation of the control unit 82. Fig. 4 shows an example of the panorama display screen. Here, there are provided a display space 85c that displays a panoramic image of the plurality of first photography images 610a, and a display space 85d which is used to check a display position of the panoramic image of the display space 85c. Note that, the display space 85d displays the second photography image 520a, and the display position of the panoramic image is indicated by the mark 611.

Further, scroll bars 86a and 86b, which change the display position of the panoramic image, are provided in the vertical and horizontal directions of the display space 85c. When the scroll bars 86a and 86b are slid by the operation of the control unit 82, the image processing unit 83 changes the display position of the panoramic image, which is displayed on the display space 85c, on the basis of the input signal, and moves the position of the mark 611 of the second photography image 520a in accordance with the display position of the panoramic image. In such a manner, the tester checks the panoramic image at a prescribed position of the fundus image while checking the position of the mark 611 of the second photography image 520a (the fundus image) of the display space 85d.

Note that, the display position of the panoramic image may be changed by moving the mark 611 (or the reticle) of the display space 85d in the second photography image 520a through the operation of the control unit 82.

In such a manner, by using the photography position information, it is possible to accurately stitch the photography images (the first photography images) with small view angles to the second photography image with a large photography area, and thus the panoramic image of the first photography images are appropriately formed. Further, by displaying the marks indicating the photography positions of the first photography images on the moving image of the second photography image, it is possible to easily photograph portions necessary for the panorama photography while checking the photography positions of the first photography images of the fundus. Furthermore, by displaying the photography history of the first photography images on the second photography image, the tester is prevented from forgetting capture of the first photography images.

Further, in the above description, both of the observation image (the moving image) of the second photography image and the photography image (still image) of the first photography image and the second fundus image stored in the storage unit 81 are simultaneously displayed on the same screen of the monitor 85. However, the observation image and the photography image may be displayed to be switched.

Fig. 5 shows a modified example of the display screen of the monitor 85. For example, in a state where the observation image and the mark 610 are displayed on the entire monitor 85, by moving the photography position of the first photography image 610a indicated by the mark 610 as described above, the still images of the first photography image 610a and the second photography image 520a are repeatedly photographed. In this case, the image processing unit 83 displays the photography completion mark 610b of the first photography image 610a on the second photography image 520 in accordance with photography completion of the first photography image 610a, thereby assisting the tester to easily view the photography completion position of the first photography image.

At this time, the image processing unit 83 repeatedly performs stitching processing as described above whenever the first photography image 610a is photographed. After the photographing of the panoramic image is completed, when the display of the monitor 85 is changed to the photography image through the operation of the control unit 82, the panoramic image is completely formed by the image processing unit 83 in advance. Hence, the entire panoramic image is displayed on the entire monitor 85. As described above, when the observation image and the photography image are displayed to be switched, the display of the monitor 85 becomes quite visible.

In the above description, the position information of the fundus indicated by the mark 610 is acquired as the photography position information, and the first photography images 610a are stitched, but the invention is not limited to this. For example, combination of coordinates of the monitor 85 and the movement position information acquired by the tracking may be used as the photography position information. In addition, the movement position information is calculated on the basis of the detection result of the tracking unit 300 mentioned above and the information (information of a driving mechanism not shown in the drawing, angular information, and the like) of the scanning section 20 of the first photographing unit 100. Alternatively, a detector used only for acquiring the movement position information may be provided as the position information acquisition section.

For example, when the origin point is set to the center of the monitor 85, the control unit 80 calculates the coordinates of the display position (the center position) of the mark 610 with reference to the origin point, acquires the movement position information based on the tracking, and stores them as the photography position information of the first photography image in the storage unit 81.

Further, position information of the provided fixation target of the fixation target optical system 260 is used in the photography position information at the time of forming the panoramic image of the first photography image 610a. In this case, the position information of the provided fixation target is stored together with the first photography image 610a in the storage unit 81. For example, when the position of the provided fixation target is sequentially changed so as to thereby change the direction of the line of sight of the eye with respect to the optical axis, the photography position of the fundus is changed, and the first photography images 610a are photographed at different photography positions of the fundus. On the other hand, the image processing unit 83 determines the position, at which the first photography images on the second photography image 520 are stitched on the basis of the position of the provided fixation target, on the basis of the above-mentioned correlation, thereby forming the panoramic image.

In this case, it is preferable that the fixation target projected onto the patient's eye from the fixation target optical system 260 should be formed to be thin to the extent that the patient's eye is able to be in visual contact with the fixation target. Further, a mark corresponding to the position of the provided fixation target is displayed on the second photography image 520 of the display section 85a. With such a configuration, the tester is able to guide the line of sight of the patient's eye in more minute steps by smoothly moving the position of the provided fixation target indicated by the mark, and is able to smoothly (continuously) form a panoramic image using the first photography images.

Further, when the fixation target is used in the photography position information, it is the premise that the patient's eye is correctly in visual contact with the fixation target. Note that, on the basis of the position of the center checked from the second photographing image 520, it can be determined whether the visual fixation of the patient's eye is normalized.

Note that, the above-mentioned fixation target optical system 260 is a polarizing member that moves the photography position relative to the eye.

Further, the photography position information may include combination of the position of the provided fixation target and the coordinates of the mark 610 for the characteristic portion of the second photography image 520. For example, the tester moves the fundus photography position indicated by the mark 610 with reference to a certain specific position of the provided fixation target, thereby acquiring the plurality of first photography images 610a. Note that, the acquired first photography image is stored in the storage unit 81 together with the position information of the provided fixation target and the coordinate position information of the mark 610.

When photographing of the first photography image at a certain position of the provided fixation target 610 is completed, the position of the provided fixation target is changed, and the plurality of first photography images 610a are photographed at different portions of the fundus. In such a manner, when a panoramic image is formed in a wider area, the stitching positions of the first photography images are more correctly specified by using the plurality of photography position information pieces.

Note that, since distortion in photography tends to occur when the first photography image 610a is photographed at the peripheral portion of the patient's eye through the guidance of the fixation target, image processing of correcting the distortion of the first photography image 610a may be performed.

Further, when the ophthalmic photographing apparatus (main body) is configured to be able to be tilted (tiltable and swingable) to the patient's eye, the panorama photographing can be performed in a state where the entire apparatus is tilted to the reference optical axis of the patient's eye. Hence, it is possible to obtain the second photography image in a wider area than the photography view angle of the second photographing unit 200. In this case, the tilt angle information is used in the photography position information, and thus the stitching positions of the photography images are determined.

Fig. 6 shows a schematic external view of the ophthalmic photographing apparatus according to a modified example. Here, the above-mentioned optical system and control system are built in a photographing unit 503, and a driving unit 506 enables the photographing unit 503 to be tilted about two horizontal and vertical axes when the pupil position (rotation center) of the patient's eye E is set as a reference (axis). In addition, the driving unit 506 is connected with an angle sensor 506a that calculates the tilt angle of the photographing unit 503 by sensing the driving amount of the driving unit, and the driving unit 506 and the angle sensor 506a are individually connected to the control unit 80.

With such a configuration, by driving the driving unit through the operation of the control unit 82, the tilt angle of the photographing unit 503 with respect to the patient's eye E is adjusted. Alternatively, in a state where the photography area on the fundus is set in advance, the tilt angle of the photographing unit may be automatically adjusted by the driving of the driving unit 506. Thereby, it is possible to change the photography position of the second photography image of the fundus. On the other hand, the control unit causes the storage unit 81 to store the driving amount of the driving unit 503, which is sensed by the angle sensor 506a, as photography position information together with the second photography image. In such a manner, on the basis of the tilt angle of the photographing unit 503, the photography position of the second photography image on the fundus is specified, and thus the second photography image is obtained in a wider area by forming a panoramic image.

In this case, also the panoramic image of the first photography images may be formed. For example, in a state where approximate alignment between the fundus and the photographing unit 503 is performed through the driving of the driving unit 506, the fine alignment of the photography position based on the mark 610 is repeated, and the plurality of first photography images are acquired. Then, through the image processing of the image processing unit 83, the panoramic image is formed in a wider area by using the first photography images.

Further, a detection portion, which is used to detect a scanning angle, may be provided in the scanning section 20, and the detection result of the detection portion may be used in the photography position information. Besides, various kinds of information for specifying the photography position of the first photography image 610a or the second photography image on the fundus may be used in the photography position information.

The present invention can be applied to a fundus photographing apparatus including an optical system that photographs the fundus image of the patient's eye E and an optical system that is able to photograph the fundus image at a visual-cell level. For example, in the case of photographing the anterior eye part of the patient's eye, even when corneal endothelial cells are photographed to obtain a high-magnification photography image, by adopting the configuration of the present invention, a panoramic image of the corneal endothelial cells is accurately formed, and thus the anterior eye part is appropriately observed.

Further, the first photography images may be stitched without using the second photography image. For example, in the same manner as described above, the correlation of the first photography images adjacent to each other is calculated, and the stitching positions are determined, thereby forming the panoramic image of the first photography images without the second photography image. It is preferable that, for the determination of the stitching positions, the position correction information should be considered as described above.

Further, the above description was given of the example in which the position correction information is stored together with the first photography image. Besides, by individually storing the first photography image and the position correction information (for example, the position information of the scanning section 20) and associating the information pieces with each other at the time of forming the panoramic image, the stitching positions of the first photography images may be determined.

Further, the configuration of the present invention may be applied to the mydriatic fundus photographing apparatus that performs fluorescent photographing of the fundus. In this case, when the high-magnification photography image at a cellular level is obtained in the fluorescent photographing, by forming the panoramic image as described above, an image with a wider view angle is obtained.

## Claims

1. An ophthalmic photographing apparatus for photographing an examinee's eye (E), the apparatus comprising:
a first photographing unit (100) that includes a first light source (1) for irradiating the examinee's eye (E), which is visually fixed by using a fixation target, with illumination light and photographs the examinee's eye (E) illuminated by the first light source (1) to obtain a first photography image of the examinee's eye;
a second photographing unit (200) that includes a second light source (76) for irradiating the examinee's eye (E) with illumination light and photographs the examinee's eye illuminated by the second light (76) source in a wider area than a photography view angle of the first photographing unit (100);
a photography area setting unit configured to move a photography area, which is photographed by the first photographing unit, relative to the examinee's eye which is photographed by the second photographing unit;
a photography position information acquisition unit configured to acquire photography position information of the first photography image of the examinee's eye from the photography area setting unit;
a storage unit (81) configured to store the first photography image and the photography position information acquired by the photography position information acquisition unit in association with each other;
a display unit (85) configured to display the first photography image which is obtained by the first photographing unit and a second photography image which is obtained by the second photographing unit; and
a display control unit (83) configured to synthesize a first mark (610b) which represents a photographing position of the first photography image photography-completed and stored in the storage unit (81), and a second mark (610) which represents a photographing position of a new first photography image to be obtained by the first photographing unit (100), on the second photography image or a photography image of the examinee's eye (E), which is photographed with a view angle the same as that of the second photography image, through image processing, so as to display the synthesized image on the display unit (85), wherein the photography area setting unit moves the photography area which is photographed by the first photographing unit and defined by the second mark.

2. The ophthalmic photographing apparatus according to claim 1, wherein the first photographing unit (100) is configured to obtain a photography image which has a higher magnification than the second photography image.

3. The ophthalmic photographing apparatus according to claim 2, wherein the display control unit (83) further uses the position information to stitch a plurality of the first photography images, of which photographing positions obtained by the first photographing unit (100) are different, to each other through image processing to display the images as one image on the display unit (85).

4. The ophthalmic photographing apparatus according to any one of claims 1 to 3, wherein
the first photographing unit (100) includes:
the first light source (1);
an illumination optical system including a scanning section (20) for scanning the illumination light from the first light source (1) on a fundus of the examinee;
a wavefront sensor (73) configured to receive at least a part of the light, which is reflected from the fundus illuminated by the first light source (1) or a light source different from the first light source (1), so as to detect wavefront aberration of the examinee's eye (E);
a wavefront compensation section including a wavefront compensation device (72) which compensates the wavefront aberration on the basis of a detection result of the wavefront sensor (73); and
a photographing optical system configured to receive the light, which is reflected from the fundus illuminated by the illumination optical system , in a state where the wavefront compensation section performs wavefront compensation on the light, so as to obtain the first photography image.

5. The ophthalmic photographing apparatus according to claim 4 further comprising:
a setting unit configured to set predetermined photographed area for acquiring the plurality of the first photography images of which the photographing positions are different from that of the second photography image displayed on the display unit (85); and
a controller (82) configured to continuously acquire the plurality of the first photography images of which the photographing positions are different by controlling the first photographing unit (100) and the scanning section (20) so as to obtain the first photography images from the predetermined photographed areas set by the setting unit.

6. The ophthalmic photographing apparatus according to any one of claims 1 to 5, further comprising a position detection unit configured to detect time-varying positional deviation of a prescribed portion of the fundus obtained by the photographing of the second photographing unit (200).

7. The ophthalmic photographing apparatus according to any one of claims 1 to 6, further comprising a tracking unit (300) configured to maintain a photographing optical axis with respect to the examinee's eye (E) in a prescribed relationship on the basis of positional deviation information obtained by the position detection unit.

8. The ophthalmic photographing apparatus according to any one of claims 1 to 7, wherein the photographing position information is at least one of position information of the first photography image relative to the second photography image, presentation position information of the fixation target, and photographing position information of the apparatus relative to the examinee's eye (E).

9. The ophthalmic photographing apparatus according to any one of claims 1 to 8, wherein the first photographing unit (100) performs at least one processing of integration processing, averaging processing, and selection processing on the plurality of the photography images which are acquired at the same photographing location.

## Patentansprüche

1. Ophthalmische Fotografiervorrichtung zum Fotografieren eines Auges eines Probanden (E), wobei die Vorrichtung umfasst:
eine erste Fotografiereinheit (100), die eine erste Lichtquelle (1) zum Beleuchten des Auges eines Probanden (E) umfasst, die unter Verwendung eines Fixierungsziels visuell mit Beleuchtungslicht fixiert wird, und das von der ersten Lichtquelle (1) beleuchtete Auge des Probanden (E) zum Erhalten eines ersten fotografischen Bildes des Auges des Probanden fotografiert;
eine zweite Fotografiereinheit (200), die eine zweite Lichtquelle (76) zum Beleuchten des Auges des Probanden (E) mit Beleuchtungslicht umfasst, und das von der zweiten Lichtquelle (76) beleuchtete Auge des Probanden in einem größeren Bereich als einem fotografischen Blickwinkel der ersten Fotografiereinheit (100) fotografiert;
eine Fotografierbereich-Einstelleinheit, die zum Bewegen eines Fotografierbereichs, der von der ersten Fotografiereinheit fotografiert wird, relativ zum Auge des Probanden, das von der zweiten Fotografiereinheit fotografiert wird, konfiguriert ist;
eine Fotografierposition-Informationserfassungseinheit, die zum Erfassen von Fotografierpositionsinformationen des ersten fotografischen Bildes des Auges des Probanden von der Fotografierbereich-Einstelleinheit konfiguriert ist;
eine Speichereinheit (81), die zum Speichern des ersten fotografischen Bildes und der von der Fotografierposition-Informationserfassungseinheit erfassten Fotografierpositionsinformationen in Verbindung miteinander konfiguriert ist;
eine Anzeigeeinheit (85), die zum Anzeigen des ersten fotografischen Bildes, das von der ersten Fotografiereinheit erhalten wird, und eines zweiten fotografischen Bildes, das von der zweiten Fotografiereinheit erhalten wird, konfiguriert ist; und
eine Anzeigesteuereinheit (83), die zum Synthetisieren einer ersten Markierung (610b), die eine Fotografierposition des ersten fotografischen Bildes darstellt, das fotografisch-fertiggestellt und in der Speichereinheit (81) gespeichert ist, und einer zweiter Markierung (610), die eine Fotografierposition eines neuen ersten fotografischen Bildes darstellt, das von der ersten Fotografiereinheit (100) auf dem zweiten fotografischen Bild oder einem fotografischen Bild des Auges (E) des Probanden erhalten werden soll, das mit einem gleichen Blickwinkel wie dem des zweiten fotografischen Bildes fotografiert wurde, durch eine Bildverarbeitung konfiguriert ist, um das synthetisierte Bild auf der Anzeigeeinheit (85) anzuzeigen, wobei die Fotografierbereich-Einstelleinheit den Fotografierbereich bewegt, der von der ersten Fotografiereinheit fotografiert wird und durch die zweite Markierung definiert ist.

2. Ophthalmische Fotografiervorrichtung nach Anspruch 1, wobei die erste Fotografiereinheit (100) zum Erhalten eines fotografischen Bildes konfiguriert ist, das eine höhere Vergrößerung als das zweite fotografische Bild aufweist.

3. Ophthalmische Fotografiervorrichtung nach Anspruch 2, wobei die Anzeigesteuereinheit (83) ferner die Positionsinformationen zum Zusammenfassen einer Vielzahl der ersten fotografischen Bilder, von denen sich die von der ersten Fotografiereinheit (100) erhaltenen Fotografierpositionen durch eine Bildverarbeitung voneinander unterscheiden, zum Anzeigen der Bilder als ein Bild auf der Anzeigeeinheit (85) verwendet.

4. Ophthalmische Fotografiervorrichtung nach einem der Ansprüche 1 bis 3, wobei
die erste Fotografiereinheit (100) umfasst:
die erste Lichtquelle (1);
ein optisches Beleuchtungssystem mit einem Abtastabschnitt (20) zum Abtasten des Beleuchtungslichts von der ersten Lichtquelle (1) auf einem Fundus des Probanden;
einen Wellenfrontsensor (73), der zum Aufnehmen zumindest eines Teils des Lichts, das von dem von der ersten Lichtquelle (1) beleuchteten Fundus oder einer von der ersten Lichtquelle (1) unterschiedlichen Lichtquelle reflektiert wird, zum Erfassen einer Wellenfrontaberration des Auges des Probanden (E) konfiguriert ist;
einen Wellenfrontkompensationsabschnitt mit einer Wellenfrontkompensationsvorrichtung (72), welche die Wellenfrontaberration basierend auf einem Erfassungsergebnis des Wellenfrontsensors (73) kompensiert; und
ein fotografisches optisches System, das zum Aufnehmen des Lichts, das vom durch das optische Beleuchtungssystem beleuchteten Fundus reflektiert wird, in einem Zustand konfiguriert ist, bei dem der Wellenfrontkompensationsabschnitt eine Wellenfrontkompensation des Lichts zum Erhalten des ersten fotografischen Bildes durchführt.

5. Ophthalmische Fotografiervorrichtung nach Anspruch 4, ferner umfassend:
eine Einstelleinheit, die zum Einstellen eines vorbestimmten fotografierten Bereichs zum Erfassen der Vielzahl der ersten fotografischen Bilder konfiguriert ist, deren Fotografierpositionen sich von denen des auf der Anzeigeeinheit (85) angezeigten zweiten fotografischen Bildes unterscheiden; und
eine Steuereinheit (82), die zum kontinuierlichen Erfassen der Vielzahl der ersten fotografischen Bilder, deren Fotografierpositionen unterschiedlich sind, durch Steuern der ersten Fotografiereinheit (100) und des Abtastabschnitts (20) zum Erhaltender ersten fotografischen Bilder aus den vorbestimmten fotografierten Bereichen konfiguriert ist, die von der Einstelleinheit eingestellt wurden.

6. Ophthalmische Fotografiervorrichtung nach einem der Ansprüche 1 bis 5, ferner umfassend eine Positionserfassungseinheit, die zum Erfassen einer zeitlich veränderlichen Positionsabweichung eines vorgeschriebenen Abschnitts des Fundus konfiguriert ist, der durch das Fotografieren der zweiten Fotografiereinheit (200) erhalten wurde.

7. Ophthalmische Fotografiervorrichtung nach einem der Ansprüche 1 bis 6, ferner umfassend eine Nachführeinheit (300), die zum Beibehalten einer fotografischen optischen Achse in Bezug auf das Auge des Probanden (E) in einer vorgeschriebenen Beziehung basierend auf den von der Positionserfassungseinheit erhaltenen Positionsabweichungsinformationen konfiguriert ist.

8. Ophthalmische Fotografiervorrichtung nach einem der Ansprüche 1 bis 7, wobei die Fotografierpositionsinformationen Positionsinformationen des ersten fotografischen Bildes relativ zum zweiten fotografischen Bild, Präsentationspositionsinformationen des Fixierungsziels und/oder Fotografierpositionsinformationen der Vorrichtung relativ zum Auge des Probanden (E) sind.

9. Ophthalmische Fotografiervorrichtung nach einem der Ansprüche 1 bis 8, wobei die erste Fotografiereinheit (100) eine Verarbeitung einer Integrationsverarbeitung, einer Mittelwertbildungsverarbeitung und/oder einer Auswahlverarbeitung an der Vielzahl der fotografischen Bilder durchführt, die am gleichen fotografischen Standort erfasst wurden.

## Revendications

1. Appareil photographique ophtalmique pour photographier l'oeil d'une personne examinée (E), l'appareil comprenant :
une première unité de photographie (100) qui comporte une première source de lumière (1) pour irradier l'oeil de la personne examinée (E), qui est fixé visuellement en utilisant une cible de fixation, avec une lumière d'éclairage et photographie l'oeil de la personne examinée (E) éclairé par la première source de lumière (1) pour obtenir une première image de photographie de l'oeil de la personne examinée ;
une deuxième unité de photographie (200) qui comporte une deuxième source de lumière (76) pour irradier l'oeil de la personne examinée (E) avec une lumière d'éclairage et photographie l'oeil de la personne examinée éclairé par la deuxième source de lumière (76) dans une zone plus large qu'un angle de vue photographique de la première unité de photographie (100) ;
une unité de réglage de zone de photographie configurée pour déplacer une zone de photographie, qui est photographiée par la première unité de photographie, par rapport à l'oeil de la personne examinée qui est photographié par la deuxième unité de photographie ;
une unité d'acquisition d'informations de position de photographie configurée pour acquérir des informations de position de photographie de la première image de photographie de l'oeil de la personne examinée à partir de l'unité de réglage de zone de photographie ;
une unité de stockage (81) configurée pour stocker la première image de photographie et les informations de position de photographie acquises par l'unité d'acquisition d'informations de position de photographie en association les unes avec les autres ;
une unité d'affichage (85) configurée pour afficher la première image de photographie qui est obtenue par la première unité de photographie et une deuxième image de photographie qui est obtenue par la deuxième unité de photographie ; et
une unité de commande d'affichage (83) configurée pour synthétiser une première marque (610b) qui représente une position de photographie de la première image de photographie, dont la photographie est achevée et stockée dans l'unité de stockage (81), et une deuxième marque (610) qui représente une position de photographie d'une nouvelle première image de photographie devant être obtenue par la première unité de photographie (100), sur la deuxième image de photographie ou une image de photographie de l'oeil de la personne examinée (E), qui est photographiée avec un angle de vue identique à celui de la deuxième image de photographie, par traitement d'image, afin d'afficher l'image synthétisée sur l'unité d'affichage (85), où l'unité de réglage de zone de photographie déplace la zone de photographie qui est photographiée par la première unité de photographie et définie par la deuxième marque.

2. Appareil photographique ophtalmique selon la revendication 1, dans lequel la première unité de photographie (100) est configurée pour obtenir une image de photographie qui a un grossissement supérieur à celui de la deuxième image de photographie.

3. Appareil photographique ophtalmique selon la revendication 2, dans lequel l'unité de commande d'affichage (83) utilise en outre les informations de position pour assembler une pluralité des premières images de photographie, dont les positions de photographie obtenues par la première unité de photographie (100) sont différentes, les unes avec les autres par traitement d'image pour afficher les images comme une seule image sur l'unité d'affichage (85).

4. Appareil photographique ophtalmique selon l'une quelconque des revendications 1 à 3, dans lequel
la première unité de photographie (100) comporte :
la première source de lumière (1) ;
un système optique d'éclairage comportant une section de balayage (20) pour balayer la lumière d'éclairage provenant de la première source de lumière (1) sur le fond d'oeil de la personne examinée ;
un capteur de front d'onde (73) configuré pour recevoir au moins une partie de la lumière, qui est réfléchie par le fond d'oeil éclairé par la première source de lumière (1) ou une source de lumière différente de la première source de lumière (1), afin de détecter une aberration de front d'onde de l'oeil de la personne examinée (E) ;
une section de compensation de front d'onde comportant un dispositif de compensation de front d'onde (72) qui compense l'aberration de front d'onde sur la base d'un résultat de détection du capteur de front d'onde (73) ; et
un système optique de photographie configuré pour recevoir la lumière, qui est réfléchie par le fond d'oeil éclairé par le système optique d'éclairage, dans un état où la section de compensation de front d'onde effectue une compensation de front d'onde sur la lumière, afin d'obtenir la première image de photographie.

5. Appareil photographique ophtalmique selon la revendication 4, comprenant en outre :
une unité de réglage configurée pour régler une zone photographiée prédéterminée afin d'acquérir la pluralité des premières images de photographie dont les positions de photographie sont différentes de celle de la deuxième image de photographie affichée sur l'unité d'affichage (85) ; et
un dispositif de commande (82) configuré pour acquérir en continu la pluralité des premières images de photographie dont les positions de photographie sont différentes en commandant la première unité de photographie (100) et la section de balayage (20) afin d'obtenir les premières images de photographie à partir des zones photographiées prédéterminées réglées par l'unité de réglage.

6. Appareil photographique ophtalmique selon l'une quelconque des revendications 1 à 5, comprenant en outre une unité de détection de position configurée pour détecter un écart de position variable dans le temps d'une partie prescrite du fond d'oeil obtenue par la photographie de la deuxième unité de photographie (200).

7. Appareil photographique ophtalmique selon l'une quelconque des revendications 1 à 6, comprenant en outre une unité de suivi (300) configurée pour maintenir un axe optique de photographie par rapport à l'oeil de la personne examinée (E) dans une relation prescrite sur la base d'informations d'écart de position obtenues par l'unité de détection de position.

8. Appareil photographique ophtalmique selon l'une quelconque des revendications 1 à 7, dans lequel les informations de position de photographie sont au moins des informations parmi des informations de position de la première image de photographie par rapport à la deuxième image de photographie, des informations de position de présentation de la cible de fixation et des informations de position de photographie de l'appareil par rapport à l'oeil de la personne examinée (E).

9. Appareil photographique ophtalmique selon l'une quelconque des revendications 1 à 8, dans lequel la première unité de photographie (100) effectue au moins un traitement parmi un traitement d'intégration, un traitement de calcul de moyenne et un traitement de sélection sur la pluralité des images de photographie qui sont acquises au même emplacement de photographie.
